Europäisches Patentamt

(19) European Patent Office

Office européen des brevets

(11) Publication number: **0 241 818 B1**

## EUROPEAN PATENT SPECIFICATION

(45) Date of publication of patent specification:
30.01.91 Bulletin 91/05

(51) Int. Cl.⁵: **A61K 47/00**

(21) Application number: 87104907.8

(22) Date of filing: 02.04.87

(54) pH buffering method.

(30) Priority: 15.04.86 JP 86787/86

(43) Date of publication of application:
21.10.87 Bulletin 87/43

(45) Publication of the grant of the patent:
30.01.91 Bulletin 91/05

(84) Designated Contracting States:
CH DE ES FR GB IT LI

(56) References cited:
DE-A- 2 718 697
DE-A- 3 321 200
FR-M- 2 924
FR-M- 6 065
US-A- 3 553 148

(73) Proprietor: Kao Corporation
1-14-10, Nihonbashi Kayaba-cho
Chuo-ku Tokyo (JP)

(72) Inventor: Kurosaki, Tomihiro
1465, Tannowa Misaki-cho
Sennan-gun Osaka-fu (JP)
Inventor: Imamura, Takashi
1450, Nishihama
Wakayama-shi Wakayama-ken (JP)

(74) Representative: Wächtershäuser, Günter, Dr.
Tal 29
D-8000 München 2 (DE)

EP 0 241 818 B1

## Description

This invention relates to a buffering method in which a pH of a solution containing metal ions, which would otherwise form insoluble metal salts with phosphoric acid salts, is buffered without formation of any insoluble metal salts.

Phosphoric acid salts have the capability of buffering pH and have wide utilization in various fields of industries as a pH buffering agent. In view of the fact that phosphoric acid salts have the function of pH buffering in the living body, they are important pH buffering agents.

In the field of certain products including food and medicines which are taken into the living body orally, intravenously or percutaneously, phosphoric acid salts are widely used as a pH buffering agent from the standpoints of a pH buffering range, safety and cost. On the other hand, in the specific fields of food, medicines, cosmetics and fragrance materials, it is frequently experienced to add alkaline metal ions such as Ca, Mg, and, transition metal ions so as to enhance the functional properties of the products.

However, these metal ions react with phosphoric acid salts to form insoluble metal salts, causing the pH buffering ability to lower and an undesirable phenomenon such as of turbidity to occur. This involves a problem of considerably lowering the values of the products such as food, medicines, cosmetics and fragrance materials.

The present inventors made intensive studies to solve the above problem. As a result, it was found that salts of phosphoric esters such as salts of sugar phosphoric esters and glycerophosophoric acid salts have a good capability of pH buffering without formation of any insoluble metal salts in a solution in which such metal ions are present which will form insoluble metal salts by reaction with phosphoric acid salts.

More particularly, the present invention provides a pH buffering method for a solution in which metal ions are present which form insoluble metal salts by reaction with phosphoric acid salts, the method characterized by adding one or more salts of phosphoric esters selected from the group consisting of salts of sugar phosphoric esters and glycerophosphoric acid salts.

Fig. 1 is a graphical representation of titration curves of buffering solutions of Examples 1 to 4 ;
Fig. 2 is a graphical representation of pH titration curves of buffering solutions of Examples 5 to 8 ;
Fig. 3 is a graphical representation of pH titration curves of buffering solutions of Example 9 ; and
Fig. 4 is a graphical representation of pH titration curves of buffering solutions of Comparative Examples 1 to 6.

The salts of phosphoric esters used in the present invention are salts of phosphoric esters with monosaccharides, disaccharides and polysaccharides, and salts of glycerophosphoric esters. Examples of the salts include salts between glucose-1-phosphate, glucose-6-phosphate, mannose-6-phosphate, galactose-6-phosphate, fructose-6-phosphate, glucose-1,6-diphosphate, fructose-1,6-diphosphate, alpha-glycerophosphoric acid, beta-glycerophosphoric acid and Na, K, Ca, Mg, $NH_4$, amines, alkanolamines and amino acids. Preferable examples include Na, K, Ca, Mg, Fe and arginine salts of glucose-1-phosphate, glucose-6-phosphate, fructose-6-phosphate, fructose-1,6-diphosphate and beta-glycerophosphoric acid.

Metal ions forming insoluble metal salts by reaction with phosphoric acid salts may be alkaline earth metals such as Ca, Mg, Ba and, Zn, Ti, Al, Cr, Mn, Pb, Sn, Cu and Ni. As a matter of course, two or more metal ions may coexist.

The concentration of metal ions forming insoluble metal salts with phosphoric acid salts present in a solution is not critical. For instance, even through metal ions are present in large amounts such that their molar concentration exceeds a molar concentration of a phosphoric acid salt, the resultant metal salt of the phosphoric ester is soluble in water and the solution is transparent, showing a satisfactory pH buffering capability.

The concentration of a salt of a phosphoric ester used in the present invention may vary depending on the degree of the pH buffering capability required and is thus not critical.

The pH buffering range varies depending on the types of acid component and base component constituting a pH buffering system. When a salt of a phosphoric ester is used as both an acid component and a base component, an effective pH buffering range is from 4 to 8, most preferably from 5 to 7. On the other hand, when a salt of a phosphoric ester is used as either an acid component or a base component and a component other than the phosphoric ester salt is used a counter base or acid component, the pH may be buffered in an acidic range below 4 or in a basic range over 8. The present invention is not limited to a specific buffering range of pH.

The solution in which metal ions are present may comprise any compounds not impeding the pH-buffering capability of the phosphoric ester salt, e.g., water-soluble solvents, surface active agents, sugars, lipids, amino acids and proteins.

The present invention provides a method for pH buffering of a solution in which metal ions forming insoluble metal salts by reaction with phosphoric acid salts are present, which method comprising adding a salt of a phosphoric ester as a pH buffering agent where the pH is buffered without formation of any insoluble metal salts. This method can be widely utilized in the fields of food, medicines, cosmetics, fragrance, materials including, more particularly, drinks, nourishing drinks, transfusions, eye drops, oral hygenic articles and bath agents.

Examples and comparative examples are described to illustrate the invention.

## Example 1

1 liter of ion-exchanged water was added to 0.111 g of $CaCl_2$ to prepare an aqueous solution with a $Ca^{2+}$ ion concentration of 0.001 mol/l.

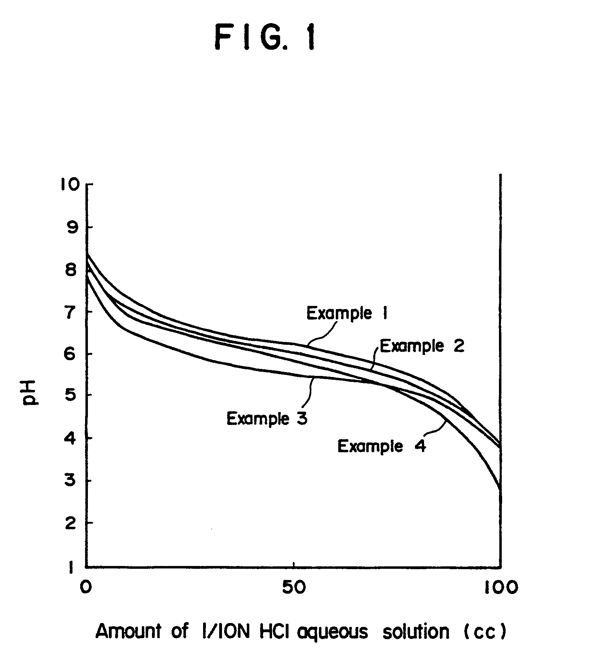

3.80 g of dipotassium glucose-1-phosphate·$2H_2O$ (purity 98%, 0.01 mol) was added to and mixed with the solution. The resultant solution was found to be transparent with a pH of 8.4. A 1/10N HCl aqueous solution was further added to the solution for pH titration. The pH titration curve is shown in Fig. 1. The solution had a pH buffering range of 5 to 7. During and after addition of 100 cc of a 1/10N HCl aqueous solution, the solution was transparent. The solution after addition of 50 cc of a 1/10N HCl aqueous solution was left as transparent on storage at 25°C for 1 week and no changes were observed.

## Example 2

1 liter of ion-exchanged water was added to 11.11 g of $CaCl_2$ to prepare an aqueous solution with a $Ca^{2+}$ ion concentration of 0.01 mol/l.

3.80 g of dipotassium glucose-1-phosphate·$2H_2O$ (purity 98%, 0.01 mol) was added to and mixed with the solution. The resultant aqueous solution was found to be transparent with a pH of 8.3. A 1/10N HCl aqueous solution was further added to the solution for pH titration. The pH titration curve is shown in Fig. 1. The solution had a pH buffering range of 5 to 7. During and after addition of 100 cc of a 1/10N HCl aqueous solution, the solution was transparent. The solution after addition of 50 cc of a 1/10N HCl aqueous solution was left as transparent on storage at 25°C for 1 week and no changes were observed.

## Example 3

1 liter of ion-exchanged water was added to 11.1 g of $CaC_2$ to prepare an aqueous solution with a $CaCl^{2+}$ ion concentration of 0.1 mol/l.

3.80 g of dipotassium glucose-1-phosphate·$2H_2O$ (purity 98%, 0.01 mol) was added to and mixed with the solution. The resultant aqueous solution was found to be transparent with a pH of 8.0. A 1/10N HCl aqueous solution was further added to the solution for pH titration. The pH titration curve is shown in Fig. 1. The solution had a pH buffering range of 5 to 7. During and after addition of 100 cc of a 1/10N HCl aqueous solution, the solution was transparent. The solution after addition of 50 cc of a 1/10N HCl aqueous solution was left as transparent on storage at 25°C for 1 week and no changes were observed.

## Example 4

1 liter of ion-exchanged water was added to 1.11 g of $CaCl_2$ to prepare an aqueous solution with $Ca^{2+}$ ion concentration of 0.01 mol/l.

3.10 g of disodium glucose-1-phosphate (purity 98%, 0.01 mol) was added to and mixed with the solution. The resultant aqueous solution was found to be transparent with a pH of 8.4. A 1/10N HCl aqueous solution was further added to the solution for pH titration. the pH titration curve is shown in Fig. 1. The solution had a pH buffering range of 5 to 7. During and after addition of 100 cc of a 1/10N HCl aqueous solution, the solution was transparent. The solution after addition of 50 cc of a 1/10N HCl aqueous solution was left as transparent on storage at 25°C for 1 week and no changes were observed.

## Example 5

1 liter of ion-exchanged water was added to 1.11 g of $CaCl_2$ to prepare an aqueous solution with a$CA^{2+}$ ion concentration of 0.01 mol/l.

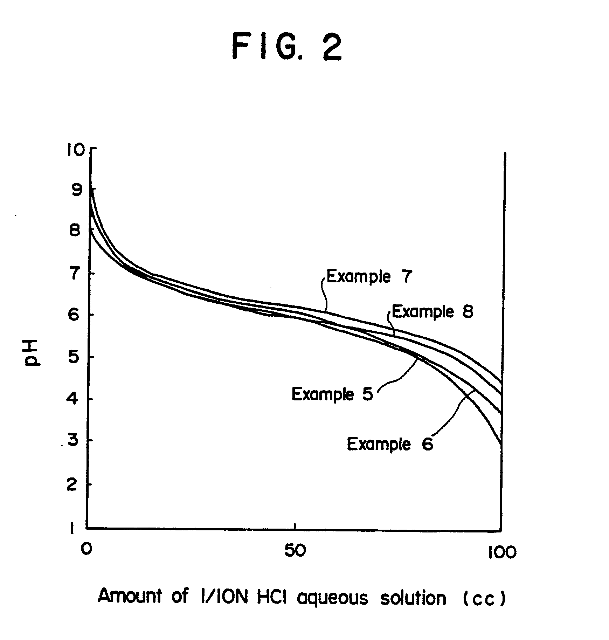

3.10 g of disodium glucose-1-phosphate (purity 98%, 0.01 mol) was added to and mixed with the solution. The resultant aqueous solution was found to be transparent with a pH of 9.4. A 1/10N HCl aqueous solution was further added to the solution for pH titration. The pH titration curve is shown in Fig. 2. The solution had a pH buffering range of 5 to 7. During and after addition of 100 cc of a 1/10N HCl aqueous solution, the solution was transparent. The solution after addition of 50 cc of a 1/10N HCl aqueous solution was left as transparent on storage at 25°C for 1 week and no changes were observed.

## Example 6

1 liter of ion-exchanged water was added to 1.11 g of $CaCl_2$ to prepare an aqueous solution with a $Ca^{2+}$ ion concentration of 0.01 mol/l.

3.56 g of disodium fructose-6-phosphate·$2.5H_2O$ (purity 98%, 0.01 mol) was added to and mixed with the solution. The resultant aqueous solution was found to be transparent with a pH of 8.5. A 1/10N HCl aqueous solution was further added to the solution for pH titration. The pH titration curve is shown in Fig. 2. The solution had a pH buffering range of 5 to 7. During and after addition of 100 cc of a 1/10N HCl aqueous solution, the solution was transparent. The solution after addition of 50 cc of a 1/10N HCl aqueous solution was left as transparent on storage at 25°C for 1 week and no changes were observed.

## Example 7

1 liter of ion-exchanged water was added to 1.11 g of $CaCl_2$ to prepare an aqueous solution with a $Ca^{2+}$

ion concentration of 0.01 mol/l.

3.12 g of disodium beta-glycerophosphate·5H$_2$O (purity 98%, 0.01 mol) was added to and mixed with the solution. The resultant aqueous solution was found to be transparent with a pH of 8.5. A 1/10N HCl aqueous solution was further added to the solution for pH titration. The pH titration curve is shown in Fig. 2. The solution had a pH buffering range of 5 to 7. During and after addition of 100 cc of a 1/10N HCl aqueous solution, the solution was transparent. The solution after addition of 50 cc of a 1/10N HCl aqueous solution was left as transparent on storage at 25°C for 1 week and no changes were observed.

## Example 8

1 liter of ion-exchanged water was added to 2.03 g of MgCl$_2$·6H$_2$O to prepare an aqueous solution with a Mg$^{2+}$ ion concentration of 0.01 mol/l.

3.80 g of dipotassium glucose-1-phosphate·2H$_2$O (purity 98%, 0.01 mol) was added to and mixed with the solution. The resultant aqueous solution was found to be transparent with a pH of 8.2. A 1/10N HCl aqueous solution was further added to the solution for pH titration. The pH titration curve is shown in Fig. 2. The solution had a buffering range of 5 to 7. During and after addition of 100 cc of a 1/10N HCl aqueous solution, the solution was transparent. The solution after addition of 50 cc of a 1/10N HCl aqueous solution was left as transparent on storage at 25°C for 1 week and no changes were observed.

## Example 9

1 liter of ion-exchanged water was added to 2.88 g of ZnSO$_4$·7H$_2$O to prepare an aqueous solution with a Zn$^{2+}$ ion concentration of 0.01 mol/l.

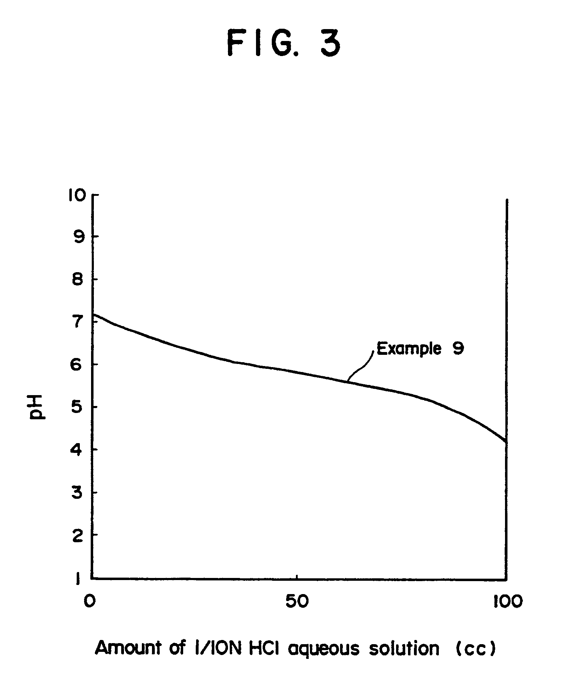

3.80 g of dipotassium glucose-1-phosphate·2H$_2$O (purity 98%, 0.01 mol) was added to and mixed with the solution. The resultant aqueous solution was found to be transparent with a pH of 7.1. A 1/10N HCl aqueous solution was further added to the solution for pH titration. The pH titration curve is shown in Fig. 3. The solution had a pH buffering range of 5 to 7. During and after addition of 100 cc of a 1/10N HCl aqueous solution, the solution was transparent. The solution after addition of 50 cc of a 1/10N HCl aqueous solution was left as transparent on storage at 25°C for 1 week and no changes were observed.

## Comparative Example 1

1 liter of ion-exchanged water was added to 0.111 g of CaCl$_2$ to prepare an aqueous solution with a Ca$^{2+}$ ion concentration of 0.001 mol/l.

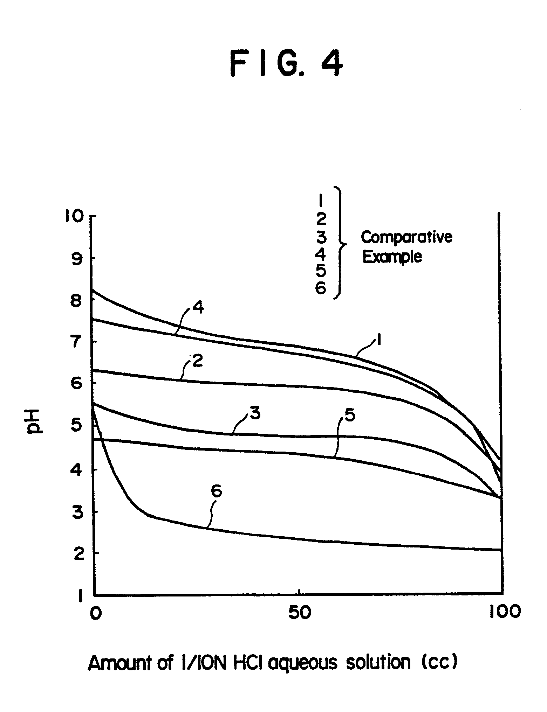

1.74 g of K$_2$HPO$_4$ (0.01 mol) was added to and mixed with the solution. The resultant aqueous solution was turbid with a pH of 8.2. A 1/10N HCl aqueous solution was further added to the solution for pH titration. The pH titration curve is shown in Fig. 4. The solution had a pH buffering range of 6 to 8. During and after addition of 50 cc of a 1/10N HCl aqueous solution, the solution remained turbid, without showing any changes on storage at 25°C for 1 week.

## Comparative Example 2

1 liter of ion-exchanged water was added to 1.11 g of CaCl$_2$ to prepare an aqueous solution with a Ca$^{2+}$ ion concentration of 0.01 mol/l.

1.74 g of K$_2$HPO$_4$ (0.01 mol) was added to and mixed with the solution. The resultant aqueous solution was in a very turbid state with a pH of 6.3. A 1/10N HCl aqueous solution was further added to the solution for pH titration. The pH titration curve is shown in Fig. 4. The solution had a pH buffering range of 5 to 6. During and after addition of 50 cc of a 1/10N HCl aqueous solution, the solution remained turbid, without showing any changes on storage at 25°C for 1 week.

## Comparative Example 3

1 liter of ion-exchanged water was added to 1.11 g of CaCl$_2$ to prepare an aqueous solution with a Ca$^{2+}$ ion concentration of 0.1 mol/l.

1.74 g of K$_2$HPO$_4$ (0.01 mol) was added to and mixed with the solution. The resultant aqueous solution was in a very turbid state with a pH of 5.5. A 1/10N HCl aqueous solution was further added to the solution for pH titration. The pH titration curve is shown in Fig. 4. The solution had a pH buffering range of 4 to 5. During and after addition of 50 cc of a 1/10N HCl aqueous solution, the solution remained turbid, without showing any changes on storage at 25°C for 1 week.

## Comparative Example 4

1 liter of ion-exchanged water was added to 2.03 g of MgCl$_2$·6H$_2$O to prepare an aqueous solution with a Mg$^{2+}$ ion concentration of 0.01 mol/l.

1.74 g of K$_2$HPO$_4$ (0.01 mol) was added to and mixed with the solution. The resultant aqueous solution was in a turbid state with a pH of 7.5. A 1/10N HCl aqueous solution was further added to the solution for pH titration. The pH titration curve is shown in Fig. 4. The solution had a pH buffering range of 6 to 7. During and after addition of 50 cc of a 1/10N HCl aqueous solution, the solution remained turbid, without showing any changes on storage at 25°C for 1 week.

## Comparative Example 5

1 liter of ion-exchanged water was added to 2.88 g of ZnSO$_4$·7H$_2$O to prepare an aqueous solution with

a $Zn^{2+}$ ion concentration of 0.01 mol/l.

1.74 g of $K_2HPO_4$ (0.01 mol) was added to and mixed with the solution. The resultant aqueous solution was in a turbid state with a pH of 4.6. A 1/10N HCl aqueous solution was further added to the solution for pH titration. The pH titration curve is shown in Fig. 4. The solution had a pH buffering range of 3 to 5. During and after addition of 50 cc of a 1/10N HCl aqueous solution, the solution remained turbid, without showing any changes on storage at 25°C for 1 week.

**Comparative Example 6**

1 liter of ion-exchanged water was added to 1.11 g of $CaCl_2$ to prepare an aqueous solution with a $Ca^{2+}$ ion concentration of 0.01 mol/l. The solution had a pH of 5.8.

A 1/10N HCl aqueous solution was further added to a solution for pH titration. The pH titration curve is shown in Fig. 4. The solution had a pH buffering range of 2 to 3. During and after the addition of the 1/10N HCl aqueous solution, the solution was transparent, without showing any changes on storage at 25°C for 1 week.

**Claims**

1. A pH buffering method for a solution which contains metal ions usually forming an insoluble metal salt with a phosphoric acid salt, characterized in that one or more salts of phosphoric esters selected from salts of sugar phosphoric esters and glycerophosphoric acid salts are added.

**Ansprüche**

1. Verfahren zur pH-Pufferung für eine Lösung, welche Metallionen enthält, die gewöhnlich ein unlösliches Metallsalz mit einem Phosphorsäuresalz bilden, dadurch gekennzeichnet, daß ein oder mehrere Salze von Phosphorsäureestern, ausgewählt aus Salzen von Zuckerphosphorsäureestern und Glycerophosphorsäuresalzen, zugesetzt werden.

**Revendications**

1. Procédé de tamponnage du pH pour une solution qui contient des ions métalliques formant habituellement un sel de métal insoluble avec un sel d'acide phosphorique, caractérisé par le fait qu'on ajoute un ou plusieurs sels d'esters phosphoriques choisis parmi les sels d'esters phosphoriques de sucres et les sels d'acide glycérophosphorique.

# FIG. 1

Example 1

Example 2

Example 3

Example 4

pH

Amount of 1/10N HCl aqueous solution (cc)

# FIG. 2

Amount of 1/10N HCl aqueous solution (cc)

# FIG. 3

Amount of I/ION HCI aqueous solution (cc)

# F I G. 4

Amount of 1/10N HCl aqueous solution (cc)